# FASCICULE DE BREVET EUROPEEN

(11) **EP 2 714 728 B1**
(45) Date de publication et mention de la délivrance du brevet: **03.05.2017**
(21) Numéro de dépôt: 12729672.1
(22) Date de dépôt: 25.05.2012
(51) Int. Cl.: C07K 14/46, C12Q 1/68

(54) **AMORCES UNIVERSELLES ET LEUR UTILISATION POUR LA DÉTECTION ET L'IDENTIFICATION D'ESPÈCES DE BATRACIENS/POISSONS**
UNIVERSELLE PRIMER UND IHRE VERWENDUNG FÜR DEN NACHWEIS UND DIE IDENTIFIZIERUNG VON AMPHIBIEN/FISCHARTEN
UNIVERSAL PRIMERS AND THE USE THEREOF FOR THE DETECTION AND IDENTIFICATION OF AMPHIBIA/FISH SPECIES

(30) Priorité: 26.05.2011 FR 1154608
(43) Date de publication de la demande: 09.04.2014
(73) Titulaire: Universite Joseph Fourier (Grenoble 1), 38041 Grenoble (FR); Centre National de la Recherche Scientifique (C.N.R.S.), 75794 Paris Cedex 16 (FR)
(72) Inventeur: TABERLET, Pierre, F-38660 La Terrasse (FR); COISSAC, Eric, F-73110 Arvillard (FR); RIAZ, Tiayyba, F-38000 Grenoble (FR)
(74) Mandataire: Agasse, Stéphane
(86) Numéro de dépôt international: PCT/FR2012/000213
(87) Numéro de publication internationale: WO 2012/160278

(56) Documents cités:
- WO-A2-2008/056325
- GB-A- 2 374 597
- TAKASHI KITANO ET AL: "Two universal primer sets for species identification among vertebrates", INTERNATIONAL JOURNAL OF LEGAL MEDICINE, SPRINGER, BERLIN, DE, vol. 121, no. 5, 15 juillet 2006 (2006-07-15), pages 423-427, XP019541912, ISSN: 1437-1596
- PEREIRA F ET AL: "Analysis of inter-specific mitochondrial DNA diversity for accurate species identification", INTERNATIONAL CONGRESS SERIES, EXCERPTA MEDICA, AMSTERDAM, NL, vol. 1288, 1 avril 2006 (2006-04-01), pages 103-105, XP025082698, ISSN: 0531-5131, DOI: 10.1016/J.ICS.2005.09.125 [extrait le 2006-04-01]
- MARTÍN IRENE ET AL: "Real-time polymerase chain reaction detection of fishmeal in feedstuffs", JOURNAL OF AOAC INTERNATIONAL, AOAC INTERNATIONAL, ARLINGTON, VA, US, vol. 93, no. 6, 1 novembre 2010 (2010-11-01), pages 1768-1777, XP009155801, ISSN: 1060-3271

### SEQUENCE LISTING

<110> UNIVERSITE JOSEPH FOURIER (GRENOBLE 1) CNRS
<120> Amorces universelles et leur utilisation pour la détection et l'identification d'espèces batraciens/poissons
<130> BR075260/NJO/CCU
<160> 52
<170> PatentIn version 3.5
<210> 1
   <211> 17
   <212> DNA
   <213> Artificial Sequence
<220>
   <223> primer
<400> 1
   acaccgcccg tcacyct 17
<210> 2
   <211> 23
   <212> DNA
   <213> Artificial sequence
<220>
   <223> primer
<400> 2
   gtayacttac catgttacga ctt 23
<210> 3
   <211> 20
   <212> DNA
   <213> Artificial Sequence
<220>
   <223> primer
<400> 3
   cttccggtac acttaccatg 20
<210> 4
   <211> 17
   <212> DNA
   <213> Artificial Sequence
<220>
   <223> sequence complementary to a primer
<400> 4
   agrgtgacgg gcggtgt 17
<210> 5
   <211> 23
   <212> DNA
   <213> Artificial Sequence
<220>
   <223> sequence complementary to a primer
<400> 5
   aagtcgtaac atggtaagtr tac 23
<210> 6
   <211> 20
   <212> DNA
   <213> Artificial Sequence
<220>
   <223> sequence complementary to a primer
<400> 6
   catggtaagt gtaccggaag 20
<210> 7
   <211> 30
   <212> DNA
   <213> Artificial Sequence
<220>
   <223> primer
<400> 7
   tcaccctcct caagtatact tcaaaggaca 30
<210> 8
   <211> 20
   <212> DNA
   <213> Artificial Sequence
<220>
   <223> primer
<400> 8
   gggattagat accccactat 20
<210> 9
   <211> 21
   <212> DNA
   <213> Artificial Sequence
<220>
   <223> primer
<400> 9
   tcaagtcctt tgggttttaag 21
<210> 10
   <211> 29
   <212> DNA
   <213> Artificial Sequence
<220>
   <223> primer
<400> 10
   ccactatgct tagccctaaa cctcaacag 29
<210> 11
   <211> 21
   <212> DNA
   <213> Artificial Sequence
<220>
   <223> primer
<400> 11
   cgagaagacc ctatggagct t 21
<210> 12
   <211> 20
   <212> DNA
   <213> Artificial Sequence
<220>
   <223> primer
<220>
   <221> misc_feature
   <222> (17)..(17)
   <223> n is a, c, g, or t
<400> 12
   cryggtcgcc ccaaccnaaa 20
<210> 13
   <211> 39
   <212> DNA
   <213> Artificial Sequence
<220>
   <223> primer
<400> 13
   tggagcttta atttattaat gcaaacagta cctaacaaa 39
<210> 14
   <211> 51
   <212> DNA
   <213> Allobates brunneus
<400> 14
   cctcttatac taaaaatagt ttttaaccaa attcagccaa tcagaagagg t 51
<210> 15
   <211> 53
   <212> DNA
   <213> Alytes obstetricans
<400> 15
   cctcaactaa ctcaaccccc taattaaaaa ctaaccagtt aacaagaaga ggc 53
<210> 16
   <211> 53
   <212> DNA
   <213> Bombina bombina
<400> 16
   cttcaactaa accaacacaa tttttaatac acaaaataag taaaagaaga ggt 53
<210> 17
   <211> 53
   <212> DNA
   <213> Bombina variegata
<400> 17
   cttcaactag aactgatata tttctaaaac ataaaacgag tacaagaaga ggt 53
<210> 18
   <211> 52
   <212> DNA
   <213> Bufo bufo
<400> 18
   cttcaaagct actaacctag tttctaacaa actaaagcat aacagaagag gc 52
<210> 19
   <211> 51
   <212> DNA
   <213> Bufo calamita
<400> 19
   cttcaaggca ctgacatagt ttttaactaa cttaagcaaa acagaagagg c 51
<210> 20
   <211> 51
   <212> DNA
   <213> Bufo viridis
<400> 20
   cttcaaagca taaacaaagt ttttaacaag tttgagcata acagaagagg c 51
<210> 21
   <211> 51
   <212> DNA
   <213> Dendrobates auratus
<400> 21
   cctcaacgct attttaaagt ttcttacaca ttttagctgc atagaagagg c 51
<210> 22
   <211> 51
   <212> DNA
   <213> Dendrobates tinctorius
<400> 22
   cctcaacgct actttaaagt ttctcacata ccttagctac atagaagagg c 51
<210> 23
   <211> 53
   <212> DNA
   <213> Discoglossus pictus
<400> 23
   cttcaacccg ccgtattcaa gtatttaaat aattttggca aaaaagaaga ggc 53
<210> 24
   <211> 51
   <212> DNA
   <213> Hyla arborea
<400> 24
   cttcaaagcc cggtattagt aattaactta acttagcaaa tcagaagagg c 51
<210> 25
   <211> 52
   <212> DNA
   <213> Hyla meridionalis
<400> 25
   cttcaaagcc taaacatcag taattaactc aaactagcac accagaagag gc 52
<210> 26
   <211> 27
   <212> DNA
   <213> Plethodon cinereus
<400> 26
   catcaaatat attactttag aagagga 27
<210> 27
   <211> 52
   <212> DNA
   <213> Pleurodeles poireti
<400> 27
   cttcaaacaa tataaaaacc ctatataaac agaaataaaa gaaagaagag gc 52
<210> 28
   <211> 53
   <212> DNA
   <213> Rana catesbeiana
<400> 28
   cttcgatagt atctcacccc gttcctaacc cactattaca ttttagaaga ggc 53
<210> 29
   <211> 54
   <212> DNA
   <213> Rana nigromaculata
<400> 29
   cttcgatagc acttcaccca ggtatttaac ccaataccgc atcttagaag aggc 54
<210> 30
   <211> 53
   <212> DNA
   <213> Rana pipiens
<400> 30
   cttcgatagt aaataatatt gtccctaacc cattatcacg ttttagaaga agc 53
<210> 31
   <211> 52
   <212> DNA
   <213> Rana temporaria
<400> 31
   cttcaatagt accccgtatg ttcctaaccc aacaccacgt tttagaagag gc 52
<210> 32
   <211> 55
   <212> DNA
   <213> Salamandra salamandra
<400> 32
   cttcaaataa tttaaaaaaa tcttaaataa ataaagtcag taagtaagaa gaggc 55
<210> 33
   <211> 53
   <212> DNA
   <213> Triturus cristatus
<400> 33
   cttcaagaac tattagatat taaataaaca aagaagaaaa aagaagaaga ggc 53
<210> 34
   <211> 52
   <212> DNA
   <213> Triturus marmoratus
<400> 34
   cttcaagcac tattttatat taaataaaca aaaagaaaaa agaagaagag gc 52
<210> 35
   <211> 63
   <212> DNA
   <213> Anguilla anguilla
<400> 35
   cctcgaataa caataaagac aattcataaa acaataagaa caaaaagagg aggcaagtcg 60
   taa 63
<210> 36
   <211> 62
   <212> DNA
   <213> Barbus barbus
<400> 36
   ccccgtcaaa atgcaccaaa atacctaatg caacagcact gacaagggga ggcaagtcgt 60
   aa 62
<210> 37
   <211> 64
   <212> DNA
   <213> Clupea harengus
<400> 37
   ccccagcgac cacccaaaaa aggtaaataa cgcaataata acagcaaggg gaggcaagtc 60
   gtaa 64
<210> 38
   <211> 63
   <212> DNA
   <213> Exocoetus volitans
<400> 38
   ccccaaaacc cctaaaaaga ttaagtaaaa ccatagatcc aataaagggg aggcaagtcg 60
   taa 63
<210> 39
   <211> 62
   <212> DNA
   <213> Gadus morhua
<400> 39
   ctccaaataa accctagata ttacctaaaa tgctttttat aataagggga ggcaagtcgt 60
   aa 62
<210> 40
   <211> 61
   <212> DNA
   <213> Hippocampus coronatus
<400> 40
   ccctaaaaca cacttaaaac taattaaaac aaaatattaa acaaggggag gcaagtcgta 60
   a 61
<210> 41
   <211> 62
   <212> DNA
   <213> Labrus merula
<400> 41
   ccccgagctt acgtatctta atacttaatg ccttataatt gcaaagggga ggcaagtcgt 60
   aa 62
<210> 42
   <211> 62
   <212> DNA
   <213> Lota Iota
<400> 42
   ctccaaatag accctaaata ttacctaaaa tgttttatat aataagggga ggcaagtcgt 60
   aa 62
<210> 43
   <211> 63
   <212> DNA
   <213> Oncorhynchus mykiss
<400> 43
   ccccaagttc aacctgtcct tctaactaag aagttaaccg aacaaagggg aggcaagtcg 60
   taa 63
<210> 44
   <211> 63
   <212> DNA
   <213> Salmo trutta
<400> 44
   ccccgagttc aattaatcct tctaactaag aagttaaccg aacaaagggg aggcaagtcg 60
   taa 63
<210> 45
   <211> 63
   <212> DNA
   <213> Salvelinus alpinus
<400> 45
   ccccaagttt aatttatcct tctaactaag aagttaacca aacaaagggg aggcaagtcg 60
   taa 63
<210> 46
   <211> 63
   <212> DNA
   <213> Salvelinus fontinalis
<400> 46
   ccccaagttt aatttatcct tctaactaag aagttaacca aacaaagggg aggcaagtcg 60
   taa 63
<210> 47
   <211> 65
   <212> DNA
   <213> Sardina pilchardus
<400> 47
   ccccaacaac tacctataaa aatgtaacta acacaatatt cgccgcaagg ggaggcaagt 60
   cgtaa 65
<210> 48
   <211> 61
   <212> DNA
   <213> Zeus faber
<400> 48
   cccctgttgg cccccaacct ttcataaaac ctttattaaa caaaggggag gcaagtcgta 60
   a 61
<210> 49
   <211> 17
   <212> DNA
   <213> Artificial Sequence
<220>
   <223> primer
<400> 49
   acaccgcccg tcaccct 17
<210> 50
   <211> 17
   <212> DNA
   <213> Artificial Sequence
<220>
   <223> primer
<400> 50
   acaccgcccg tcactct 17
<210> 51
   <211> 23
   <212> DNA
   <213> Arfificial Sequence
<220>
   <223> primer
<400> 51
   gtacacttac catgttacga ctt 23
<210> 52
   <211> 23
   <212> DNA
   <213> Artificial Sequence
<220>
   <223> primer
<400> 52
   gtatacttac catgttacga ctt 23

## Description

La présente invention concerne des oligonucléotides et leur utilisation comme amorces universelles pour la détection et l'identification d'espèces de batraciens et/ou poissons, notamment dans des substrats complexes et dégradés.

L'identification taxonomique basée sur l'analyse de l'ADN est une approche couramment utilisée aujourd'hui pour identifier des espèces au sein d'un mélange. Un consortium international nommé Barcode for Life a été créé avec comme objectif de permettre la détection d'espèces animales ou végétales à partir d'une séquence d'ADN. Pour identifier les animaux, l'approche consiste à amplifier puis à séquencer une séquence de 648 paires de base du gène mitochondrial cytochrome c oxidase 1 ("CO1") CO1 s'est montré efficace pour l'identification des oiseaux, papillons, poissons, mouches et autres groupes animaux (Hebert *et al.,* 2003). Pour les végétaux, la situation est plus complexe et deux autres séquences d'ADN chloroplastiques, *mat*K and *rbc*L*,* ont été proposées (Hollingsworth *et al.,* 2009). Il s'avère néanmoins que les fragments choisis selon cette méthode sont de longueur trop longue (supérieure à 500 nucléotides) pour être utilisés dans des substrats dégradés. Or, dans de très nombreuses situations, les substrats à analyser contiennent de l'ADN dégradé, avec souvent des fragments de moins de 100 nucléotides. C'est le cas notamment des échantillons collectés dans l'environnement (sol, eau, fèces) ou des matrices utilisées dans l'industrie, par exemple les farines animales ou les plats industriels de l'industrie alimentaire.

Une solution pour la détection et l'identification spécifique consiste à analyser des fragments d'ADN informatifs de petite taille (Poinar *et al*., 1998; Willersley *et al.*, 2003 ; Willersley *et al.,* 2007). Des amorces ont ainsi été définies afin d'amplifier des fragments d'ADN végétaux plus courts dans des échantillons de sols gelés. Néanmoins, les amorces permettent d'identifier les familles mais pas les espèces végétales. En outre, les amorces n'ont pas été choisies de manière à amplifier des séquences suffisamment conservées dans le règne végétal pour permettre l'amplification de toute espèce végétale. En d'autres mots, ces amorces ne sont pas véritablement universelles.

WO 2006/024751 décrit un procédé permettant de détecter simultanément dans un échantillon de matière biologique la présence éventuelle de matières biologiques par réaction de polymérisation en chaîne (PCR) puis hybridation avec des sondes. Les amorces décrites sont très dégénérées (pratiquement chaque codon comporte un nucléotide dégénéré) et manquent par conséquent de spécificité pour l'amplification de l'ADN de vertébrés.

Le brevet EP1797201 propose des oligonucléotides permettant la détection et l'identification de végétaux dans des substrats complexes ou dégradés car la région amplifiée est à la fois courte et très variable. Plus précisément, la région amplifiée correspond à une région variable de l'intron du gène chloroplastique *trn*L du tabac. A cet égard, référence est faite également à l'article de Taberlet *et al.,* 2007.

Les amorces décrites dans ce brevet sont spécifiques des espèces végétales et elles ne permettent pas l'identification et la détection des espèces de batraciens et/ou poissons dans des substrats complexes ou dégradés.

Takashi et al. (2006) décrivent une méthode utilisant des oligonucléotides universels pour l'identification d'espèces basée sur la PCR. La méthode décrite permet l'amplification de fragments de taille allant de 215 à 244 pb et permet d'identifier différents type de vertébrés allant de l'homme, l'oiseau, les reptiles les batraciens et les poissons. L'utilisation de ces amorces inadaptée pour l'amplification de fragments à partir d'ADN dégradé.

Pereira et al. (2006) décrivent une méthode visant à l'identification d'espèces de Mammifères basée sur l'utilisation de dix-sept paires d'amorces pour amplifier des fragments d'ADN mitochondrial de type 12S d'une taille de moins de 100 pb.Martin et al. (2010) décrivent une méthode pour identifier la présence de farine de poissons dans les aliments à destination des animaux par une méthode d'amplification par PCR de fragments d'une taille de 87 pb à partir du gène de l'ARN mitochondrial 12S.

La présente invention propose de nouveaux oligonucléotides et leurs utilisations comme amorces universelles pour l'identification et la détection des espèces batraciens et/ou poissons. Ces amorces permettent à la fois d'amplifier une région courte (moins de 95 paires de base), une région très variable entre les espèces batraciens et/ou poissons et une région présentant dans le même temps des régions flanquantes très conservées permettant l'amplification à l'aide une seule et unique paire d'amorces. Ainsi ces amorces peuvent être utilisées dans des mélanges complexes et dégradés, par exemple des échantillons de sols, de fèces et d'eau. De telles amorces trouvent notamment des applications dans l'analyse des plats industriels susceptibles de contenir des mélanges de batraciens et/ou poissons, mais également pour l'analyse des régimes alimentaires des carnivores à partir des fèces.

Plus précisément, ces amorces permettent d'amplifier une région 12S de l'ADN mitochondrial. Il est particulièrement intéressant de disposer d'amorces permettant l'amplification d'ADN mitochondrial car celui-ci représente une cible très accessible dans le cas de substrats dégradés. De plus, l'ADN mitochondrial est présent de manière répétée dans chaque cellule.

Les amorces universelles objet de la présente invention sont particulièrement avantageuses car elles sont extrêmement spécifiques des batraciens et/ou poissons et n'amplifient aucun autre groupe taxonomique.

### Description de l'invention

L'invention concerne une paire d'oligonucléotides, selon laquelle le premier oligonucléotide s'hybride de manière sélective à la séquence SEQ ID NO. 4 et le deuxième nucléotide s'hybride de manière sélective à la séquence SEQ ID NO. 5 ou à la séquence SEQ ID NO. 6 dans des conditions de stringences suffisantes pour l'amplification d'une région variable du gène mitochondrial 12S des batraciens et poissons par réaction de polymérisation en chaîne (PCR).

Selon un aspect de l'invention, la région amplifiée du gène mitochondrial 12S comporte moins de 95 nucléotides.

Selon un autre aspect de l'invention, le premier oligonucléotide présente une séquence nucléotidique SEQ ID NO. 1 et le deuxième oligonucléotide présente une séquence nucléotidique SEQ ID NO. 2 ou SEQ ID NO. 3.

Selon un autre aspect encore de l'invention, la région amplifiée par réaction de polymérisation en chaîne présente une séquence nucléotidique sélectionnée dans le groupe consistant en SEQ ID NOS.14-48.

La présente invention propose aussi un mélange d'amorces pour l'amplification d'une région variable du gène mitochondrial 12S des batraciens et poissons par réaction de polymérisation en chaîne (PCR) comprenant les amorces d'amplification selon SEQ ID NO.1, SEQ ID NO.2 et SEQ ID NO.3.

Selon un aspect de l'invention, le mélange d'amorces comprend en outre une amorce de blocage selon SEQ ID NO.7.

La présente invention concerne aussi un procédé d'amplification d'une région du gène mitochondrial 12S des espèces batraciens et poissons, comprenant les étapes suivantes :
a) on dispose d'un échantillon susceptible de contenir de l'ADN d'une espèce batracien et/ou poisson;
b) on réalise une réaction d'amplification en chaîne en utilisant une paire d'oligonucléotides selon l'invention ou un mélange d'amorces selon l'invention.

La présente invention concerne également un procédé de détection d'une espèce batracien et/ou poisson dans un échantillon, comprenant les étapes suivantes :
a) on dispose d'un échantillon susceptible de contenir de l'ADN d'une espèce batracien et/ou poisson;
b) on isole l'ADN total contenu dans l'échantillon;
c) on réalise une réaction d'amplification en chaîne en utilisant une paire d'oligonucléotides selon l'invention ou un mélange d'amorces selon l'invention; et
d) on détecte l'éventuelle présence d'un produit d'amplification.

La présente invention concerne aussi un procédé de détection et d'identification d'une espèce batracien et/ou poisson dans un échantillon, comprenant les étapes suivantes :
a) on dispose d'un échantillon susceptible de contenir de l'ADN d'une espèce batracien et/ou poisson;
b) on isole l'ADN total contenu dans l'échantillon ;
c) on réalise une réaction d'amplification en chaîne en utilisant une paire d'oligonucléotides selon l'invention ou un mélange d'amorces selon l'invention;
d) on détecte la présence d'un produit d'amplification ; et
e) on détermine la séquence du produit d'amplification pour identifier l'espèce batracien et/ou poisson contenue dans l'échantillon.

La présente invention propose également un kit pour la détection d'une espèce batracien et/ou poisson dans un échantillon, comprenant une paire d'oligonucléotides selon l'invention ou un mélange d'amorces selon l'invention et au moins un réactif additionnel.

Selon un aspect de l'invention, le kit comprend une autre paire d'oligonucléotides choisis parmi les paires d'amorces :
- amorces d'amplification selon SEQ ID NO.8 et SEQ ID NO.9, éventuellement avec amorce de blocage selon SEQ ID NO.10; et
- amorces d'amplification selon SEQ ID NO.11 et SEQ ID NO.12, éventuellement avec amorce de blocage selon SEQ ID NO.13.

### Description du listage de séquences

SEQ ID NO.1 : amorce BT_F
SEQ ID NO.2 : amorce B_R
SEQ ID NO.3 : amorce T_R
SEQ ID NO.4 : séquence de la région complémentaire de l'amorce BT_F
SEQ ID NO.5 : séquence de la région complémentaire de l'amorce B_R
SEQ ID NO.6 : séquence de la région complémentaire de l'amorce T_R
SEQ ID NOS.8-9 et 11-12 : paire d'amorces d'amplification additionnelles pouvant être ajoutées au kit de détection selon l'invention
SEQ ID NOS.7, 10 et 13: amorces de blocage
SEQ ID NOS.14-48 : exemples de séquences variables amplifiées de différentes espèces de batraciens et poissons
SEQ ID NOS. 49-50 : variants de l'amorce BT_F
SEQ ID NOS. 51-52 : variants de l'amorce B_R

### Paire d'oligonucléotides

La présente invention concerne donc des oligonucléotides dérivés de deux régions conservées d'un gène 12S mitochondrial présent chez les batraciens et poissons. Ces oligonucléotides peuvent être utilisés comme amorces pour l'amplification et donc la détection de l'ADN de batraciens et poissons dans un échantillon susceptible de contenir un tel ADN. En effet, les régions conservées dont sont dérivés les polynucléotides de la présente invention flanquent une région à la fois courte et très variable de l'ADN des batraciens et poissons, plus précisement une région courte et variable dans le gène 12S mitochondrial des batraciens et poissons. La variablité de cette région entre espèces de batraciens et poissons peut donc être utilisée pour détecter et identifier les espèces de batraciens et poissons.

Selon la présente invention, on entend par « oligonucléotide » une chaîne nucléotidique simple brin ou son complémentaire pouvant être de type ADN ou ARN, ou une chaîne nucléotidique double brin pouvant être de type ADN complémentaire ou génomique. Selon un mode réalisation, les oligonucléotides de l'invention sont de type ADN, notamment ADN double brin. Le terme « oligonucléotide » désigne également les polynucléotides modifiés. Les oligonucléotides modifiés sont par exemple des oligonucléotides conjugués à des réactifs de liaison (biotine par exemple) ou à des réactifs marqués (marqueurs fluorescents par exemple). Classiquement, les réactifs de liaison ou les réactifs marqués conjugués aux oligonucléotides facilitent la purification ou la détection de ces oligonucléotides.

Les oligonucléotides de la présente invention peuvent être préparés par synthèse chimique ou par des techniques classiques de biologie moléculaire telles que décrites par Sambrook, Fristsch et Maniatis, dans leur ouvrage intitulé "Molecular cloning: a laboratory manual", édition : Cold Spring Harbor Laboratory Press, Cold Spring Harbor, NY, 1989. Les oligonucléotides de la présente invention peuvent également être isolés ou purifiés de leur environnement naturel.

Le terme « polynucléotide » peut ici être utilisé en remplacement du terme « oligonucléotide » avec une signification équivalente.

On entend par « amorce » une courte séquence d'oligonucléotides qui, hybridée avec une matrice d'acide nucléique, permet à une polymérase d'entamer la synthèse d'un nouveau brin de l'ADN. Le brin produit à partir de l'amorce est complémentaire au brin utilisé comme matrice. Les amorces sont notamment utilisées dans les réactions de polymérisation en chaîne (PCR).

On entend par «espèce batracien» ou «espèce poisson » tout organisme faisant respectivement partie du règne batracien ou du règne poisson.

La présente invention concerne donc une paire d'oligonucléotides selon laquelle le premier oligonucléotide s'hybride de manière sélective à une région très conservée du gène mitochondrial 12S des batraciens et poissons et le deuxième nucléotide s'hybride de manière sélective à une autre région très conservée du gène mitochondrial 12S des batraciens ou à une autre région très conservée du gène mitochondrial 12S des poissons dans des conditions de stringences suffisantes pour l'amplification de la région variable du gène mitochondrial 12S des batraciens et poissons par réaction de polymérisation en chaîne (PCR), par exemple dans des conditions de forte stringence. La séquence de la première région conservée correspond à la séquence de l'amorce BT_F (SEQ ID NO. 1) et de sa séquence complémentaire (SEQ ID NO. 4). La séquence de la deuxième région conservée correspond à la séquence de l'amorce B_R (SEQ ID NO.2) et de sa séquence complémentaire (S E Q ID NO. 5). Alternativement, la séquence de la deuxième région conservée correspond à la séquence de l'amorce T_R (SEQ ID NO.3) et de sa séquence complémentaire (SEQ ID NO. 6).

A titre d'exemple, chez *Rana nigromaculata*, le premier oligonucléotide de la présente invention se trouve à la position 3525 à 3541 de la séquence NC_002805, et le second oligonucléotide de l'invention se trouve à la position 3596 à 3618 de cette même séquence. Chez *Gadus morhua,* le premier oligonucléotide de la présente invention se trouve à la position 904 à 920 de la séquence NC_002081, et le second oligonucléotide de l'invention se trouve à la position 983 à 1002 de cette même séquence.

L'homme du métier connaît les réactions d'amplification d'ADN et les conditions de stringence permettant une amplification sélective d'une séquence. L'homme du métier connait en particulier les conditions de température d'hybridation et les conditions de composition du tampon d'hybridation.

L'homme du métier pourra donc facilement définir différentes variantes des amorces BT_F (SEQ ID NO. 1) et B_R (SEQ ID NO. 2) ou T_R (SEQ ID NO. 3) en utilisant des techniques de routine. Ces variantes s'hybrident aux séquences de référence et permettent l'amplification sélective de la région variable d'intérêt de l'ADN mitochondrial 12S.

Deux variantes possible de l'amorce BT_F (SEQ ID NO. 1) sont représentées aux séquences SEQ ID NOS. 49-50. Deux variantes possibles de l'amorce B_R (SEQ ID NO.2) sont représentées aux séquences SEQ ID NOS. 51-52. Habituellement, les variations de séquence sont plutôt introduites à l'extrémité 5' des oligonucléotides pour ne pas compromettre la réaction d'amplification. Classiquement, on peut par exemple introduire des nucléotides supplémentaires à l'extrémité 5' des oligonucléotides.

Par « séquence capable de s'hybrider de manière sélective » ou « oligonucléotide capable de s'hybrider de manière sélective », on entend selon l'invention les séquences qui s'hybrident avec la séquence de référence à un niveau supérieur au bruit de fond de manière significative. Le niveau du signal généré par l'interaction entre la séquence capable de s'hybrider de manière sélective et les séquences de référence est généralement 10 fois, de préférence 100 fois plus intense que celui de l'interaction des autres séquences d'ADN générant le bruit de fond. Les conditions d'hybridation stringentes permettant une hybridation sélective sont connues de l'homme du métier. En général, la température d'hybridation et de lavage est inférieure d'au moins 5°C, par exemple 10°C, au Tm de la séquence de référence à un pH donné et pour une force ionique donnée. Typiquement, la température d'hybridation est d'au moins 30°C pour un polynucléotide de 15 à 50 nucléotides et d'au moins 60°C pour un polynucléotide de plus de 50 nucléotides. A titre d'exemple, l'hybridation est réalisée dans le tampon suivant : 6X SSC, 50 mM Tris-HCl (pH 7.5), 1 mM EDTA, 0.02% PVP, 0.02% Ficoll, 0.02% BSA, 500 µg/ml sperme de saumon dénaturé DNA. Les lavages sont par exemple réalisés successivement à faible stringence dans un tampon 2X SSC, 0,1% SDS, à moyenne stringence dans un tampon 0,5X SSC, 01% SDS et à forte stringence dans un tampon 0,1 X SSC, 0,1 %SDS. L'hybridation peut bien entendu être effectuée selon d'autres méthodes usuelles connues de l'homme du métier (voir notamment Sambrook, Fristsch et Maniatis, dans leur ouvrage intitulé "Molecular cloning: a laboratory manual", édition : Cold Spring Harbor Laboratory Press, Cold Spring Harbor, NY, 1989).

Par « stringence », on entend la rigueur des conditions opératoires (notamment la température et la force ionique) dans lesquelles se déroule une hybridation moléculaire.

Le paramètre déterminant dans la spécificité et la réversibilité de l'hybridation moléculaire est le Tm ou température de fusion (melting temperature). C'est la température à laquelle la moitié de l'ADN est sous forme monobrin et l'autre moitié sous forme double brin. Le Tm dépend de nombreux facteurs tels que la longueur du fragment d'ADN considéré, sa richesse en cytosines et guanines et la concentration en sels, notamment en ion Na du milieu réactionnel. En pratique, l'expérimentateur peut créer ou supprimer l'hybridation moléculaire en choisissant une température du milieu réactionnel inférieure, égale ou supérieure au Tm. Les conditions de forte stringence sont celles pour lesquelles la température d'hybridation et de lavage est inférieure d'au moins 5°C et jusqu'à moins 10°C au Tm de la séquence de référence à un pH donné et pour une force ionique donnée.

Par « amplification », on entend toute amplification enzymatique *in vitro* d'une séquence définie d'ADN, notamment du type réaction de polymérisation en chaîne (ou Polymerase Chain Reaction, PCR).

Les amorces selon l'invention peuvent en outre comporter des étiquettes qui vont permettre d'identifier rapidement l'appartenance de chaque séquence amplifiée à un échantillon de départ. Le terme « queue » peut être utilisée de manière équivalente, à la place du terme « étiquette » (« tags » en anglais). Les étiquettes sont de courtes séquences nucléotidiques, généralement de longueur inférieure à 10 nucléotides. Ces étiquettes sont particulièrement utiles lorsqu'il s'agit d'utiliser des séquenceurs de nouvelle génération qui permettent de séquencer près de 100 000 séquences à la fois. En effet, chaque étiquette correspondant à un échantillon de départ à analyser, on peut ainsi mélanger plusieurs produits d'amplification avant de procéder au séquençage. Ceci est avantageux car mettre en oeuvre un séquençage via un séquenceur de nouvelle génération peut s'avérer être une opération coûteuse. Dans la pratique, les amorces selon l'invention peuvent comporter un fragment nucléotidique additionnel du coté 5' spécifique de chaque échantillon. Après amplification de plusieurs échantillons, mélange des différents produits d'amplification ainsi obtenus, et séquençage, chaque séquence amplifiée est ainsi facilement assignée à un échantillon de départ. Les étiquettes constituent donc un outil particulièrement avantageux lorsqu'il s'agit de séquencer à haut débit (Binladen *et al.,* 2007).

Habituellement, l'amplification comporte des cycles successifs (généralement de 20 à 40) d'amplification, eux-mêmes composés de trois phases: après une étape de dénaturation (séparation des deux brins de la double hélice) de l'ADN, le positionnement des amorces (courtes séquences d'oligonucléotides spécifiquement choisies) en face de leurs séquences complémentaires, sur les brins d'ADN, et leur fixation sur ces cibles constitue la deuxième phase du procédé (hybridation). La phase d'extension fait intervenir une enzyme, l'ADN polymérase, qui synthétise, à partir des amorces, le brin complémentaire de celui ayant servi de matrice. La répétition de ce cycle conduit à l'amplification exponentielle du fragment d'ADN, également appelé produit d'amplification ou ADN amplifié. Selon un aspect de l'invention, l'hybridation s'effectue à une température comprise entre 45 et 65°C. Notamment, l'hybridation peut s'effectuer à une température comprise entre 45 et 60°C lorsque les amorces ne comportent pas d'étiquette. Alternativement, l'hybridation peut s'effectuer à une température comprise entre 50 et 65°C lorsque les amorces comportent une étiquette.

Selon un autre aspect, l'invention concerne une paire d'oligonucléotides, selon laquelle le premier oligonucléotide s'hybride de manière sélective à la séquence SEQ ID NO. 4 et le deuxième nucléotide s'hybride de manière sélective à la séquence SEQ ID NO. 5 dans des conditions de stringences suffisantes pour l'amplification d'une région variable du gène mitochondrial 12S de *Rana nigromaculata* dont la séquence est représentée à la SEQ ID NO.29 et qui peut être utilisée comme séquence de référence batracien.

Selon un autre aspect, l'invention concerne une paire d'oligonucléotides, selon laquelle le premier oligonucléotide s'hybride de manière sélective à la séquence SEQ ID NO. 4 et le deuxième nucléotide s'hybride de manière sélective à la séquence SEQ ID NO. 6 dans des conditions de stringences suffisantes pour l'amplification d'une région variable du gène mitochondrial 12S de *Gadus morhua* dont la séquence est représentée à la SEQ ID NO.39 et qui peut être utilisée comme séquence de référence poisson.

Selon un aspect de l'invention, la paire d'oligonucléotides permet l'amplification d'une région du gène mitochondrial 12S qui comporte moins de 95 nucléotides, en particulier moins de 90 ou moins de 80 nucléotides, amorces non comprises (c'est-à-dire sans compter la longueur des amorces). Disposer d'une paire d'amorce universelle qui permette l'amplification d'une région variable courte est très avantageuse car l'utilisation de telles amorces dans des substrats complexes ou dégradés permet la détection et si besoin l'identification des espèces dans de tels substrats.
Les amorces de la présente invention permettent de détecter et identifier plus de 4012 espèces de batraciens et poissons. Les tableaux 1 et 2 représentent une partie des séquences susceptibles d'être amplifié avec les amorces de la présente invention. Selon un aspect de la présente invention, la région amplifiée par réaction de polymérisation en chaîne présente une séquence nucléotidique sélectionnée dans le groupe consistant en l'une quelconque des SEQ ID NOS. 14-48.

La présente invention concerne également un mélange d'amorces pour l'amplification d'une région variable du gène mitochondrial 12S des vertébrés par réaction de polymérisation en chaîne (PCR) comprenant les amorces d'amplification BT_F (SEQ ID NO.1), B_R (SEQ ID NO.2) et T_R (SEQ ID NO.3) selon un aspect de l'invention, le mélange comprend en outre une amorce de blocage selon SEQ ID NO.7. Alternativement, l'invention concerne également un mélange d'amorces pour l'amplification d'une région variable du gène mitochondrial 12S des vertébrés par réaction de polymérisation en chaîne (PCR) comprenant les amorces d'amplification BT_F et B_R ou T_R et au moins une autre paire d'oligonucléotides choisies parmi les paires d'amorces :
- amorces d'amplification selon SEQ ID NO.8 et SEQ ID NO.9, éventuellement avec amorce de blocage selon SEQ ID NO.10 ; et
- amorces d'amplification selon SEQ ID NO.11 et SEQ ID NO.12, éventuellement avec amorce de blocage selon SEQ ID NO.13.

### Procédés mettant en oeuvre la paire d'oligonucléotides

La présente invention concerne également un procédé d'amplification d'une région du gène mitochondrial 12S des batraciens/poissons, comprenant les étapes suivantes :
a) on dispose d'un échantillon contenant de l'ADN de batraciens/poissons ;
b) on réalise une réaction d'amplification en chaîne en utilisant une paire d'oligonucléotides selon l'invention.

L'échantillon à partir duquel la réaction d'amplification est réalisée peut être collecté dans l'environnement. Dans ce cas, il peut par exemple s'agir d'un échantillon de sol, eau ou fèces. L'échantillon peut également provenir de l'industrie, par exemple des plats industriels (aliments transformés) de l'industrie alimentaire. L'échantillon en question peut par exemple provenir d'un produit qui a été congelé, lyophilisé ou chauffé. Un avantage du procédé selon l'invention utilisant les amorces ci-dessus décrites est que l'ADN contenu dans l'échantillon peut se trouver sous forme dégradée. Cela est principalement dû au fait que les amorces ont été choisies de manière à ce que la région amplifiée soit courte, par exemple de longueur inférieure à 95 paires de bases.

Selon un aspect de l'invention, l'hybridation s'effectue à une température comprise entre 45 et 65°C. En fonction du fait que les paires d'oligonucléotides de l'invention comporte ou non une étiquette tel que cela est décrit précédemment, les conditions de l'amplification, en particulier la température d'hybridation, peut ou non varier. Notamment, l'hybridation peut s'effectuer à une température comprise entre 45 et 60°C lorsque les amorces ne comportent pas d'étiquette. Par exemple, on peut utiliser une température de 51-53°C. Alternativement, l'hybridation peut s'effectuer à une température comprise entre 50 et 65°C lorsque les amorces comportent une étiquette. Par exemple, on peut utiliser une température de 57-58°C. L'homme du métier sait adapter la température d'hybridation aux amorces utilisées.

Le procédé d'amplification selon l'invention peut nécessiter une étape d'extraction de l'ADN avant d'effectuer l'amplification. On utilise alors par exemple pour cela un kit d'extraction disponible dans le commerce.

La présente invention propose également un procédé de détection d'une espèce de batraciens/poissons dans un échantillon. Ce procédé de détection selon l'invention comprend les étapes suivantes :
a) on dispose d'un échantillon susceptible de contenir de l'ADN d'espèces batraciens/poissons ;
b) on isole l'ADN total contenu dans l'échantillon ;
c) on réalise une réaction d'amplification en chaîne en utilisant une paire d'oligonucléotides selon l'invention ; et
d) on détecte l'éventuelle présence d'un produit d'amplification.

L'invention se rapporte aussi à un procédé de détection et d'identification d'une espèce batraciens/poissons dans un échantillon, comprenant les étapes suivantes :
a) on dispose d'un échantillon susceptible de contenir de l'ADN d'espèces batraciens/poissons ;
b) on isole l'ADN total contenu dans l'échantillon ;
c) on réalise une réaction d'amplification en chaîne en utilisant une paire d'oligonucléotides selon l'invention ;
d) on détecte la présence d'un produit d'amplification ; et
e) on détermine la séquence du produit d'amplification pour identifier l'espèce batraciens/poissons contenue dans l'échantillon.
L'étape de détermination de la séquence correspond au séquençage de celle-ci. Les méthodes et outils de séquençage sont connus de l'homme du métier et un exemple de ce qui peut être utilisé dans la présente invention est donné dans la partie expérimentale.

### Kit de détection

L'invention a également trait à un kit pour la détection d'une espèce batraciens/poissons dans un échantillon, ledit kit comprenant une paire d'oligonucléotides selon l'invention.

Selon un aspect de l'invention, le kit comprend en outre une amorce de blocage selon SEQ ID NO.7. Utiliser une amorce de blocage est utile lorsqu'il s'agit d'éviter l'amplification d'une espèce déterminée, par exemple l'homme ou la vache. Ces amorces sont choisies à partir de l'extrémité la plus variable du fragment amplifié, et de manière à ce que l'amorce de blocage se chevauche avec l'amorce d'amplification sur au moins 6 nucléotides, par exemple sur 8 nucléotides. Enfin, il est nécessaire que le Tm de l'amorce de bloquage soit nettement supérieur à celui des amorces d'amplification (au moins 5°C, par exemple 10°C).

Selon un autre aspect encore de l'invention, le kit comprend au moins une autre paire d'oligonucléotides. Cette au moins une autre paire d'oligonucléotides peut être alors utilisée à titre de vérification du contenu de l'échantillon ou à titre d'alternatives, par exemple si la paire d'oligonucléotides BT_F (SEQ ID NO. 1) et B_R (SEQ ID NO. 2) ou B_R (SEQ ID NO.3) ne permettait pas l'amplification.

Selon un autre aspect encore de l'invention, le kit comprend en outre une étiquette de moins de 10 nucléotides pour l'identification de l'échantillon de départ après séquençage du produit PCR.

Le kit selon l'invention peut également en outre contenir de manière non limitative des réactifs additionnels, par exemple une enzyme DNA polymerase, des dNTP, du Tris-HCl du KCI, du MgCl₂ ou du Bovine Serum Albumin (BSA).

### Exemples

### Exemple 1 : Validation par PCR électronique à l'aide du logiciel ecoPCR (Ficetola et al. 2010) des amorces BT_F et B_R

Cette expérimentation a été conduite avec la version 107 d'EMBL, en tolérant un nombre maximum de trois mésappariements par amorce.

Résultats : le nombre total de séquences de batraciens amplifiées est de 5501, dont 2837 séquences uniques correspondant à 2106 espèces et 357 genres.

La longueur du fragment amplifié (sans les amorces) varie entre 16 et 71 paires de bases, avec une moyenne de 50,6 paires de bases.

Le nombre moyen de mésappariements est de 0,16 pour l'amorce BT_F, et de 0,48 pour l'amorce B_R.

Discussion : Etant donné que le nombre de séquences uniques est nettement plus important que le nombre d'espèce, cela indique que la résolution de cette région permet dans la très grande majorité des cas une identification au niveau de l'espèce.

### Exemple 2 : Validation par PCR électronique à l'aide du logiciel ecoPCR (Ficetola et al. 2010) des amorces BT_F et T_R

Cette expérimentation a été conduite avec la version 107 d'EMBL, en tolérant un nombre maximum de trois mésappariements par amorce.

Résultats : le nombre total de séquences de téléostéens amplifiées est de 2742, dont 1929 séquences uniques correspondant à 1906 espèces et 357 genres.

La longueur du fragment amplifié (sans les amorces) varie entre 48 et 91 paires de bases, avec une moyenne de 63,4 paires de bases.

Le nombre moyen de mésappariements est de 0,21 pour l'amorce BT_F, et de 0,35 pour l'amorce T_R.

Discussion : Etant donné que le nombre de séquences uniques est légérement plus important que le nombre d'espèce, cela indique que la résolution de cette région permet dans la majorité des cas une identification au niveau de l'espèce.

**Tableau 1. Batraciens**

| Tableau élaboré à partir de la version 107 de la base de données EMBL | | | | |
|---|---|---|---|---|
| Nom scientifique | Numéro d'accession | Séquence correspondant à BT_F | Séquence amplifiée | Séquence correspondant à B_R |
| *Allobates brunneus* | DQ502047 | ACACCGCCCGTCACCCT | | |
| *Alytes obstetricans* | AY364340 | ACACCGCCCGTCACCCT | | |
| *Bombina bombina* | AY458591 | ACACCGCCCGTCACCCT | | |
| *Bombina variegata* | AY971143 | ACACCGCCCGTCACCCT | | |
| *Bufo bufo* | AY325988 | ACACCGCCCGTCACCCT | | |
| *Bufo calamita* | EU938400 | ACACCGCCCGTCACCCT | | |
| *Bufo viridis* | AY680267 | ACACCGCCCGTCACCCT | | |
| *Dendrobates auratus* | AY326030 | ACACCGCCCGTCACCCT | | |
| *Dendrobates tinctorius* | DQ502248 | ACACCGCCCGTCACCCT | | |
| *Discoglossus pictus* | AY364342 | ACACCGCCCGTCACCCT | | |
| | | | 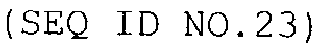 | |
| *Hyla arborea* | AY843601 | ACACCGCCCGTCACCCT | | |
| *Hyla meridionalis* | AY819370 | ACACCGCCCGTCACCCT | | |
| *Plethodon cinereus* | AY728232 | ACACCGCCCGTCACCCT | | |
| *Pleurodeles poireti* | EU880329 | ACACCGCCCGTCACCCT | | |
| *Rana catesbeiana* | AY779206 | ACACCGCCCGTCACCCT | | |
| *Rana nigromaculata* | NC_002805 | | | |
| *Rana pipiens* | AY779221 | ACACCGCCCGTCACCCT | | |
| *Rana temporaria* | AF249023 | ACACCGCCCGTCACCCT | | |
| *Salamandra salamandra* | DQ283440 | ACACCGCCCGTCACCCT | | |
| *Triturus cristatus* | DQ283441 | ACACCGCCCGTCACCCT | | |
| *Triturus marmoratus* | EU880337 | ACACCGCCCGTCACCCT | | |

**Tableau 2. Poissons téléostéens**

| Tableau élaboré à partir de la version 107 de la base de données EMBL | | | | |
|---|---|---|---|---|
| Nom scientifique | Numéro d'accession | Séquence correspondant à BT_F | Séquence amplifiée | Séquence correspondant à T_R |
| | | | | |
| *Anguilla anguilla* | AB021887 | ACACCGCCCGTCACTCT | | CTTCCGGTACACTTACCGTG |
| *Barbus barbus* | AB238965 | ACACCGCCCGTCACTCT | | CTTCCGGTACACTTACCATG |
| *Clupea harengus* | AP009133 | ATACCGCCCGTCACTCT | | CTTCCGGTATACTTACCATG |
| *Exocoetus volitans* | AP002933 | ACACCGCCCGTCACCCT | | CTTCCGGTACACTTACCATG |
| *Gadus morhua* | AM489716 | ACACCGCCCGTCACTCT | | CTTCCGGTACGCTTACCATG |
| *Hippocampus coronatus* | AB032030 | ACACCGCCCGTCACTCT | | CTTCCGGTACGCTTACCATG |
| *Labrus merula* | AJ810141 | ACACCGCCCGTCACTCT | | CTTCCGGTACACTTACCATG |
| *Lota lota* | AP004412 | ACACCGCCCGTCACTCT | | CTTCCGGTACGCTTACCATG |
| *Oncorhynchus mykiss* | AF113120 | ACACCGCCCGTCACTCT | | CTTCCGGTACACTTACCATG |
| | | | 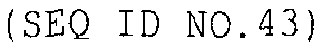 | |
| *Salmo trutta* | AM910409 | ACACCGCCCGTCACTCT | | CTTCCGGTACACTTACCATG |
| *Salvelinus alpinus* | AF154851 | ACACCGCCCGTCACTCT | | CTTCCGGTACACTTACCATG |
| *Salvelinus fontinalis* | AF154850 | ACACCGCCCGTCACTCT | | CTTCCGGTACACTTACCATG |
| *Sardina pilchardus* | AP009233 | ACACCGCCCGTCACTCT | | CTTCCGGTACACTTACCATG |
| *Zeus faber* | AP002941 | ACACCGCCCGTCACTCT | | CTTCCGGTACGCTTACCATG |

## Revendications

1. Paire d'oligonucléotides, selon laquelle le premier oligonucléotide s'hybride de manière sélective à la séquence SEQ ID NO. 4 et le deuxième nucléotide s'hybride de manière sélective à la séquence SEQ ID NO. 5 ou à la séquence SEQ ID NO. 6 dans des conditions de stringences suffisantes pour l'amplification d'une région variable du gène mitochondrial 12S des batraciens et poissons par réaction de polymérisation en chaîne (PCR).

2. Paire d'oligonucléotides selon la revendication 1, selon laquelle la région amplifiée du gène mitochondrial 12S comporte moins de 95 nucléotides.

3. Paire d'oligonucléotides selon la revendication 1 ou 2, selon laquelle le premier oligonucléotide présente une séquence nucléotidique SEQ ID NO. 1 et le deuxième oligonucléotide présente une séquence nucléotidique SEQ ID NO. 2 ou SEQ ID NO. 3.

4. Paire d'oligonucléotides selon l'une quelconque des revendications précédentes, **caractérisée en ce que** la région amplifiée par réaction de polymérisation en chaîne présente une séquence nucléotidique sélectionnée dans le groupe consistant en SEQ ID NOS.14-48.

5. Mélange d'amorces pour l'amplification d'une région variable du gène mitochondrial 12S des batraciens et poissons par réaction de polymérisation en chaîne (PCR) comprenant les amorces d'amplification selon SEQ ID NO.1, SEQ ID NO.2 et SEQ ID NO.3.

6. Mélange d'amorces selon la revendication 5, comprenant en outre une amorce de blocage selon SEQ ID NO.7.

7. Procédé d'amplification d'une région du gène mitochondrial 12S des espèces batraciens et poissons, comprenant les étapes suivantes :
a) on dispose d'un échantillon susceptible de contenir de l'ADN d'une espèce batracien et/ou poisson;
b) on réalise une réaction d'amplification en chaîne en utilisant une paire d'oligonucléotides selon l'une quelconque des revendications 1 à 4 ou un mélange d'amorces selon l'une quelconque des revendications 5-6.

8. Procédé de détection d'une espèce batracien et/ou poisson dans un échantillon, comprenant les étapes suivantes :
a) on dispose d'un échantillon susceptible de contenir de l'ADN d'une espèce batracien et/ou poisson;
b) on isole l'ADN total contenu dans l'échantillon ;
c) on réalise une réaction d'amplification en chaîne en utilisant une paire d'oligonucléotides selon l'une quelconque des revendications 1 à 4 ou un mélange d'amorces selon l'une quelconque des revendications 5-6; et
d) on détecte l'éventuelle présence d'un produit d'amplification.

9. Procédé de détection et d'identification d'une espèce batracien et/ou poisson dans un échantillon, comprenant les étapes suivantes :
a) on dispose d'un échantillon susceptible de contenir de l'ADN d'une espèce batracien et/ou poisson;
b) on isole l'ADN total contenu dans l'échantillon ;
c) on réalise une réaction d'amplification en chaîne en utilisant une paire d'oligonucléotides selon l'une quelconque des revendications 1 à 4 ou un mélange d'amorces selon l'une quelconque des revendications 5-6;
d) on détecte la présence d'un produit d'amplification ; et
e) on détermine la séquence du produit d'amplification pour identifier l'espèce batracien et/ou poisson contenue dans l'échantillon.

10. Kit pour la détection d'une espèce batracien et/ou poisson dans un échantillon, comprenant une paire d'oligonucléotides selon l'une quelconque des revendications 1 à 4 ou un mélange d'amorces selon l'une quelconque des revendications 5-6 et au moins un réactif additionnel.

11. Kit selon la revendication 10, comprenant une autre paire d'oligonucléotides choisis parmi les paires d'amorces :
- amorces d'amplification selon SEQ ID NO.8 et SEQ ID NO.9, éventuellement avec amorce de blocage selon SEQ ID NO.10; et
- amorces d'amplification selon SEQ ID NO.11 et SEQ ID NO.12, éventuellement avec amorce de blocage selon SEQ ID NO.13.

## Patentansprüche

1. Oligonukleotidpaar, wobei das erste Oligonukleotid selektiv mit der Sequenz SEQ ID Nr. 4 hybridisiert und das zweite Nukleotid selektiv mit der Sequenz SEQ ID Nr. 5 oder der Sequenz SEQ ID Nr. 6 hybridisiert, unter ausreichenden stringenten Bedingungen zur Amplifikation einer variablen Region des mitochondrialen Gens 12S von Amphibien und Fischen durch Polymerisationskettenreaktion (PCR).

2. Oligonukleotidpaar nach Anspruch 1, wobei die amplifizierte Region des mitochondrialen Gens 12S weniger als 95 Nukleotide umfasst.

3. Oligonukleotidpaar nach Anspruch 1 oder 2, wobei das erste Oligonukleotid eine Nukleotidsequenz SEQ ID Nr. 1 aufweist und das zweite Oligonukleotid eine Nukleotidsequenz SEQ ID Nr. 2 oder SEQ ID Nr. 3 aufweist.

4. Oligonukleotidpaar nach einem der vorhergehenden Ansprüche, **dadurch gekennzeichnet, dass** die durch Polymerisationskettenreaktion amplifizierte Region eine Nukleotidsequenz aufweist, die aus der Gruppe bestehend aus den SEQ ID Nrn. 14-48 ausgewählt ist.

5. Primergemisch zur Amplifikation einer variablen Region des mitochondrialen Gens 12S von Amphibien und Fischen durch Polymerisationskettenreaktion (PCR), wobei das Gemisch Amplifikationsprimer gemäß SEQ ID Nr. 1, SEQ ID Nr. 2 und SEQ ID Nr. 3 umfasst.

6. Primergemisch nach Anspruch 5, das außerdem einen Blockierungsprimer gemäß SEQ ID Nr. 7 umfasst.

7. Verfahren zur Amplifikation einer Region des mitochondrialen Gens 12S von Amphibien- und Fischspezies, wobei das Verfahren die folgenden Schritte umfasst:
a) Bereitstellen einer Probe, die möglicherweise DNA von einer Amphibien- und/oder Fischspezies enthält;
b) Durchführen einer Amplifikationskettenreaktion unter Verwendung eines Oligonukleotidpaars nach einem der Ansprüche 1 bis 4 oder eines Primergemischs nach einem der Ansprüche 5-6.

8. Verfahren zum Nachweis einer Amphibien- und/oder Fischspezies in einer Probe, wobei das Verfahren die folgenden Schritte umfasst:
a) Bereitstellen einer Probe, die möglicherweise DNA von einer Amphibien- und/oder Fischspezies enthält;
b) Isolieren der Gesamt-DNA, die in der Probe enthalten ist;
c) Durchführen einer Amplifikationskettenreaktion unter Verwendung eines Oligonukleotidpaars nach einem der Ansprüche 1 bis 4 oder eines Primergemischs nach einem der Ansprüche 5-6; und
d) Nachweisen des eventuellen Vorhandenseins eines Amplifikationsprodukts.

9. Verfahren zum Nachweis und zur Identifizierung einer Amphibien- und/oder Fischspezies in einer Probe, wobei das Verfahren die folgenden Schritte umfasst:
a) Bereitstellen einer Probe, die möglicherweise DNA von einer Amphibien- und/oder Fischspezies enthält;
b) Isolieren der Gesamt-DNA, die in der Probe enthalten ist;
c) Durchführen einer Amplifikationskettenreaktion unter Verwendung eines Oligonukleotidpaars nach einem der Ansprüche 1 bis 4 oder eines Primergemischs nach einem der Ansprüche 5-6;
d) Nachweisen des Vorhandenseins eines Amplifikationsprodukts; und
e) Bestimmen der Sequenz des Amplifikationsprodukts zum Identifizieren der Amphibien- und/oder Fischspezies, die in der Probe enthalten ist.

10. Kit zum Nachweis einer Amphibien- und/oder Fischspezies in einer Probe, das ein Oligonukleotidpaar nach einem der Ansprüche 1 bis 4 oder ein Primergemisch nach einem der Ansprüche 5-6 und mindestens ein zusätzliches Reagenz umfasst.

11. Kit nach Anspruch 10, das ein weiteres Oligonukleotidpaar umfasst, das aus den folgenden Primerpaaren ausgewählt ist:
- Amplifikationsprimer gemäß SEQ ID Nr.8 und SEQ ID Nr. 9, gegebenenfalls mit einem Blockierungsprimer gemäß SEQ ID Nr. 10; und
- Amplifikationsprimer gemäß SEQ ID Nr. 11 und SEQ ID Nr. 12, gegebenenfalls mit einem Blockierungsprimer gemäß SEQ ID Nr. 13.

## Claims

1. A pair of oligonucleotides, according to which the first oligonucleotide selectively hybridizes to the sequence SEQ ID NO. 4 and the second nucleotide selectively hybridizes to the sequence SEQ ID NO. 5 or to the sequence SEQ ID NO. 6 under sufficient stringency conditions for amplifying a variable region of the mitochondrial gene 12S of batrachians and fishes by polymerase chain reaction (PCR).

2. The pair of oligonucleotides according to claim 1, according to which the amplified region of the mitochondrial gene 12S includes lower than 95 nucleotides.

3. The pair of oligonucleotides according to claim 1 or 2, according to which the first oligonucleotide has a nucleotide sequence SEQ ID NO. 1 and the second oligonucleotide has a nucleotide sequence SEQ ID NO. 2 or SEQ ID NO. 3.

4. The pair of oligonucleotides according to any one of the preceding claims, **characterized in that** the amplified region by polymerase chain reaction has a nucleotide sequence selected from the group consisting of SEQ ID NOS.14-48.

5. A mixture of primers for amplifying a variable region of the mitochondrial gene 12S of batrachians and fishes by polymerase chain reaction (PCR) comprising the amplification primers according to SEQ ID NO. 1, SEQ ID NO. 2 and SEQ ID NO. 3.

6. The mixture of primers according to claim 5, further comprising a blocking primer according to SEQ ID NO. 7.

7. A method for amplifying a region of the mitochondrial gene 12S of the species of batrachians and fishes, comprising the following steps:
a) a sample likely to contain DNA of a batrachian and/or fish species is available;
b) performing a chain amplification reaction using a pair of oligonucleotides according to any one of claims 1 to 4 or a mixture of primers according to any one of claims 5-6.

8. A method for detecting a species of batrachian and/or fish in a sample, comprising the following steps:
a) a sample likely to contain DNA of a species of batrachian and/or fish is available;
b) isolating the total DNA contained in the sample;
c) performing a chain amplification reaction using a pair of oligonucleotides according to any one of claims 1 to 4 or a mixture of primers according to any one of claims 5- 6; and
d) detecting the possible presence of an amplification product.

9. A method for detecting and identifying a batrachian and/or fish species in a sample, comprising the following steps:
a) a sample likely to contain DNA from a batrachian and/or fish species is available;
b) isolating the total DNA contained in the sample;
c) performing a chain amplification reaction using a pair of oligonucleotides according to any one of claims 1 to 4 or a mixture of primers according to any one of claims 5-6;
d) detecting the presence of an amplification product; and
e) determining the sequence of the amplification product in order to identify the batrachian and/or fish species contained in the sample.

10. A kit for detecting a batrachian and/or fish species in a sample, comprising a pair of oligonucleotides according to any one of claims 1 to 4 or a mixture of primers according to any one of claims 5-6 and at least one additional reagent.

11. The kit according to claim 10, comprising another pair of oligonucleotides selected among the pairs of primers:
- amplification primers according to SEQ ID NO. 8 and SEQ ID NO. 9, possibly with blocking primer according to SEQ ID NO.10; and
- amplification primers according to SEQ ID NO.11 and SEQ ID NO.12, possibly with blocking primer according to SEQ ID NO.13.
